# EUROPEAN PATENT APPLICATION

(11) **EP 4 850 598 A1**
(43) Date of publication of application: **09.12.2026**
(21) Application number: 25749101.9
(22) Date of filing: 17.01.2025
(51) Int. Cl.: A61B 5/024, A61B 5/0245, A61B 5/00, A61B 7/04

(54) **METHOD AND DEVICE FOR ANALYZING ABNORMALITY OF USER STATE ON BASIS OF SMART ALL-IN-ONE HEART PATCH**

(30) Priority: 30.01.2024 KR 20240014350
(71) Applicant: Wismedical Co., Ltd, Yongin-si, Gyeonggi-do 16864 (KR)
(72) Inventor: LEE, Sung Hoon, Seoul 05649 (KR); YEO, Woon Hong, Daejeon 35246 (KR); LEE, Yoon Jae, Seoul 06601 (KR); KIM, Seong Eun, Yongin-si Gyeonggi-do 16813 (KR)
(74) Representative: HGF
(86) International application number: PCT/KR2025/001008
(87) International publication number: WO 2025/165014

(57) **Abstract**

In an embodiment, a method of providing user analysis information by a user device based on a Smart All-in-One Cardiac Patch may include a step of receiving cardiac-related signals acquired from the Smart All-in-One Cardiac Patch, a step of providing the cardiac-related signals as input to a condition monitoring learning model, a step of automatically detecting whether an abnormality exists in the user based on inference of the condition monitoring learning model, and a step of performing user information analysis based on the detection of whether an abnormality exists in the user and displaying the analyzed user information.

## Description

### TECHNICAL FIELD

The present invention relates to a method and device for analyzing abnormality in user condition based on a Smart All-in-One Cardiac Patch. More particularly, the present invention relates to a method of monitoring and managing user condition through a Smart All-in-One Cardiac Patch Device attachable to the human body.

### BACKGROUND ART

Conventional systems have been able to measure cardiac beating and analyze signals generated by the cardiac beating to identify abnormalities in a user's condition or diseases. However, since conventional systems analyze signals through specific devices provided by medical institutions or the like, a user was required to visit the medical institution and measure cardiac beating through the specific device within the medical institution to check for abnormalities in condition, presence of diseases, and the like.

However, cardiac beating can be subject to change due to various factors such as the user's physical condition and psychological state, and thus there may be limitations in identifying abnormalities in condition or diseases based on cardiac beating signals measured for a short period of time at medical institutions. In view of the foregoing, the following describes a method and device for performing real-time monitoring by being attached to the human body and measuring and transmitting cardiac beating signals in real-time, and for identifying the user's condition based thereon.

### SUMMARY OF INVENTION

### TECHNICAL PROBLEM

The present specification relates to a method and device for analyzing abnormality in user condition based on a Smart All-in-One Cardiac Patch.

The present specification relates to a method and device for building an Artificial Intelligence (AI)/Machine Learning (ML) learning model based on cardiac-related information for a plurality of users acquired based on a Smart All-in-One Cardiac Patch.

The present specification relates to a Smart All-in-One Cardiac Patch System and a method of operation thereof.

The present specification relates to a method and device for providing information regarding whether an abnormality exists in user condition by monitoring in real-time signals generated from the heart based on a Smart All-in-One Cardiac Patch Device.

### SOLUTION TO PROBLEM

In an embodiment, a method of providing user analysis information by a user device based on a Smart All-in-One Cardiac Patch may include a step of receiving cardiac-related signals acquired from the Smart All-in-One Cardiac Patch, a step of providing the cardiac-related signals as input to a condition monitoring learning model, a step of automatically detecting whether an abnormality exists in the user based on inference of the condition monitoring learning model, and a step of performing user information analysis based on the detection of whether an abnormality exists in the user and displaying the analyzed user information.

In an embodiment, the condition monitoring learning model may be obtained from a cloud, wherein the cloud receives cardiac-related signals of Smart All-in-One Cardiac Patches respectively connected with each of a plurality of user devices from the plurality of user devices, and may update the condition monitoring learning model based on the plurality of cardiac-related signals.

In an embodiment, the cloud may acquire a plurality of cardiac-related signals in real-time and may further acquire cardiac-related raw data based on a batch load, wherein the plurality of cardiac-related signals acquired in real-time and the cardiac-related raw data acquired based on the batch load may be processed in parallel and reflected in the condition monitoring learning model.

In an embodiment, the Smart All-in-One Cardiac Patch acquires cardiac-related signals based on user identification information and transmits them to a user device, and the user device records the cardiac-related signals acquired from the Smart All-in-One Cardiac Patch together with a session key, a Medium Access Control (MAC) address-based identifier of the user device, and a unique time based on the user identification information, and transmits cardiac-related information to the cloud based on the record, wherein the cardiac-related information may be transmitted to the cloud together with a signal type, a data access time, and a data modification time based on the user identification information.

In an embodiment, the cloud may derive record information excluding the user identification information from information acquired from the user device, and may encode the record information with a hexadecimal key and provide it as training data for the condition monitoring learning model based on administrator authority.

In an embodiment, the Smart All-in-One Cardiac Patch may sense at least one of Electrocardiogram (ECG), which is an electrical signal related to cardiac beating, Seismocardiogram (SCG), which is a low-frequency vibration signal, and Phonocardiogram (PCG), which is a cardiac acoustic signal, and transmit it to a user device.

In an embodiment, the user device may further acquire additional information related to the cardiac-related signals and generate cardiac-related information based on the additional information.

In an embodiment, the user device may display the analyzed user information based on an interface including user-related information and analysis information.

### ADVANTAGEOUS EFFECTS

The present specification has an effect of providing a method of analyzing abnormality in user condition based on a Smart All-in-One Cardiac Patch.

The present specification has an effect of providing a method of building an Artificial Intelligence (AI)/Machine Learning (ML) learning model based on cardiac-related information for a plurality of users acquired based on a Smart All-in-One Cardiac Patch.

The present specification has an effect of providing a Smart All-in-One Cardiac Patch System and a method of operation thereof.

The present specification has an effect of providing information regarding whether an abnormality exists in user condition by monitoring in real-time signals generated from the heart based on a Smart All-in-One Cardiac Patch Device.

The technical problem to be solved by the present specification is not limited to the foregoing, and may be expanded to various aspects that can be derived from the embodiments of the invention described hereinafter.

### BRIEF DESCRIPTION OF DRAWINGS

FIG. 1 is a diagram illustrating an example of an operating environment of a system in accordance with an embodiment of the present specification.
FIG. 2 is a block diagram for explaining an internal configuration of a computing device 200 in accordance with an embodiment of the present specification.
FIG. 3 is a diagram showing a Smart All-in-One Cardiac Patch Device and a plurality of user devices in accordance with an embodiment of the present specification.
FIG. 4 is a diagram showing a Smart All-in-One Cardiac Patch System in accordance with an embodiment of the present specification.
FIG. 5 is a diagram showing a method of providing analysis information by a user device in accordance with an embodiment of the present specification.
FIG. 6 is a diagram showing the structure of a Smart All-in-One Cardiac Patch in accordance with an embodiment of the present specification.
FIG. 7 is a diagram showing an internal structure of a Smart All-in-One Cardiac Patch in accordance with an embodiment of the present specification.
FIG. 8 is a diagram showing a method of detecting whether an abnormality exists based on machine learning in accordance with an embodiment of the present specification.
FIG. 9 is a diagram showing a method of analyzing signals obtained based on user condition information in accordance with an embodiment of the present specification.
FIG. 10 is a diagram showing a method of training a learning model on a cloud basis in accordance with an embodiment of the present specification.
FIG. 11 is a diagram showing a method of updating a condition monitoring learning model in accordance with an embodiment of the present specification.
FIG. 12 is a diagram showing a method of displaying cardiac-related information by a user device in accordance with an embodiment of the present specification.
FIG. 13 is a diagram showing a Smart All-in-One Cardiac Patch System in accordance with an embodiment of the present specification.
FIG. 14 is a diagram showing a method of performing a user information security operation in accordance with an embodiment of the present specification.
FIG. 15 is a diagram showing a Smart All-in-One Cardiac Patch method and device in accordance with an embodiment of the present specification.
FIG. 16 is a diagram showing a method of providing cardiac-related information to a user in accordance with an embodiment of the present invention.

### DESCRIPTION OF EMBODIMENTS

In describing embodiments of the present specification, detailed description regarding known configurations or functions is omitted in cases where it is determined that such description may obscure the gist of the embodiments of the present specification. Parts in the drawings that are unrelated to the description of embodiments of the present specification are omitted, and similar reference numerals are used for similar parts.

In embodiments of the present specification, when a component is "connected," "coupled," or "connected" to another component, this includes not only direct connection relationships but also indirect connection relationships in which another component exists therebetween. Additionally, when a component "includes" or "has" another component, unless specifically stated otherwise, this does not exclude other components but means that another component may be further included.

In embodiments of the present specification, terms such as first, second, and the like are used only for the purpose of distinguishing one component from another component, and unless specifically mentioned, do not limit order or importance between components. Accordingly, within the scope of embodiments of the present specification, a first component in one embodiment may be referred to as a second component in another embodiment, and similarly, a second component in one embodiment may be referred to as a first component in another embodiment.

In embodiments of the present specification, distinct components are for the purpose of clearly describing the characteristics of each, and do not necessarily mean that the components are separate. That is, a plurality of components may be integrated to form a single hardware or software unit, or a single component may be distributed to form a plurality of hardware or software units. Accordingly, even without specific mention, embodiments integrated or distributed in this manner are also included within the scope of embodiments of the present specification.

In the present specification, the network may be a concept that includes both wired and wireless networks. In this case, the network may refer to a communication network through which data exchange can be performed between devices and systems and between devices, and is not limited to a specific network.

The embodiments described in the present specification may have an aspect that is entirely hardware, partially hardware and partially software, or entirely software. In the present specification, "unit," "device," or "system" and the like refer to computer-related entities such as hardware, a combination of hardware and software, or software. For example, a unit, module, device, or system and the like in the present specification may be a running process, processor, object, executable, thread of execution, program, and/or computer, but is not limited thereto. For example, both an application running on a computer and the computer itself may correspond to a unit, module, device, or system and the like of the present specification.

Additionally, in the present specification, a device may be a mobile device such as a smartphone, tablet personal computer, wearable device, and Head Mounted Display (HMD), or may be a stationary device such as a personal computer or a home appliance with display function. Additionally, as an example, a device may be an in-vehicle cluster or an Internet of Things (IoT) device. That is, in the present specification, a device may refer to devices capable of running applications, and is not limited to a specific type. Hereinafter, for convenience of description, a device on which an application runs is referred to as a device.

In the present specification, the communication method of the network is not limited, and connections between respective components may not be connected via the same network method. The network may include not only a communication method utilizing a communication network (for example, mobile communication network, wired internet, wireless internet, broadcasting network, satellite network, etc.) but also short-range wireless communication between devices. For example, the network may include all communication methods through which objects can network with each other, and is not limited to wired communication, wireless communication, 3G, 4G, 5G, or other methods. For example, wired and/or wireless network may be Local Area Network (LAN), Metropolitan Area Network (MAN), Global System for Mobile Communications (GSM), Enhanced Data rates for GSM Evolution (EDGE), High Speed Downlink Packet Access (HSDPA), Wideband Code Division Multiple Access (W-CDMA), Code Division Multiple Access (CDMA), Time Division Multiple Access (TDMA), Bluetooth, Zigbee, Wi-Fi, Voice over Internet Protocol (VoIP), LTE Advanced, IEEE 802.16m, WirelessMAN-Advanced, HSPA+, 3GPP Long Term Evolution (LTE), Mobile WiMAX (IEEE 802.16e), UMB (formerly EV-DO Rev. C), Flash-OFDM, iBurst and MBWA (IEEE 802.20) systems, HIPERMAN, Beam-Division Multiple Access (BDMA), Wi-MAX (World Interoperability for Microwave Access), and ultrasonic communication, selected from a group consisting of one or more communication methods, but is not limited thereto.

Components described in various embodiments do not necessarily mean essential components, and some may be optional components. Accordingly, embodiments constituted by a subset of the components described in the embodiments are also included within the scope of embodiments of the present specification. Additionally, embodiments further including other components in addition to the components described in various embodiments are also included within the scope of embodiments of the present specification.

Hereinafter, embodiments of the present specification are described in detail with reference to the drawings.

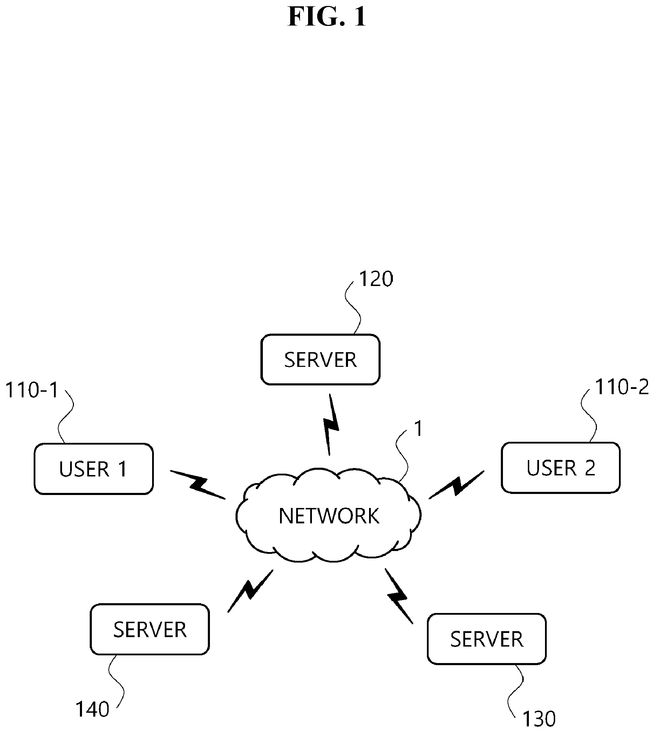

FIG. 1 is a diagram illustrating an example of an operating environment of a system in accordance with an embodiment of the present specification. Referring to FIG. 1, one or more user devices (110-1, 110-2) and one or more servers (120, 130, 140) are connected via a network (1). FIG. 1 is an example for the purpose of explaining the invention, and the number of user devices or servers is not limited as shown in FIG. 1.

One or more user devices (110-1, 110-2) may be stationary terminals or mobile terminals implemented as computer systems. One or more user devices (110-1, 110-2) may include, for example, a smartphone, a mobile phone, a navigation device, a computer, a notebook computer, a digital broadcasting terminal, a Personal Digital Assistant (PDA), a Portable Multimedia Player (PMP), a tablet personal computer, a game console, a wearable device, an Internet of Things (IoT) device, a Virtual Reality (VR) device, an Augmented Reality (AR) device, and the like. As an example, in the embodiments, a user device (110) may refer to one of various physical computer systems capable of substantially communicating with other servers (120-140) via the network (1) using wireless or wired communication methods.

Each server may be implemented as a computer device or a plurality of computer devices that communicate with one or more user devices (110-1, 110-2) via the network (1) to provide commands, codes, files, contents, services, and the like. For example, a server may be a system that provides respective services to one or more user devices (110-1, 110-2) that access via the network (1). As a more specific example, a server may provide, through an application as a computer program installed and executed on one or more user devices (110-1, 110-2), a service intended by the application (for example, information provision, etc.) to one or more user devices (110-1, 110-2). As another example, a server may distribute files for installation and execution of the application described above to one or more user devices (110-1, 110-2) and provide corresponding services by receiving user input information.

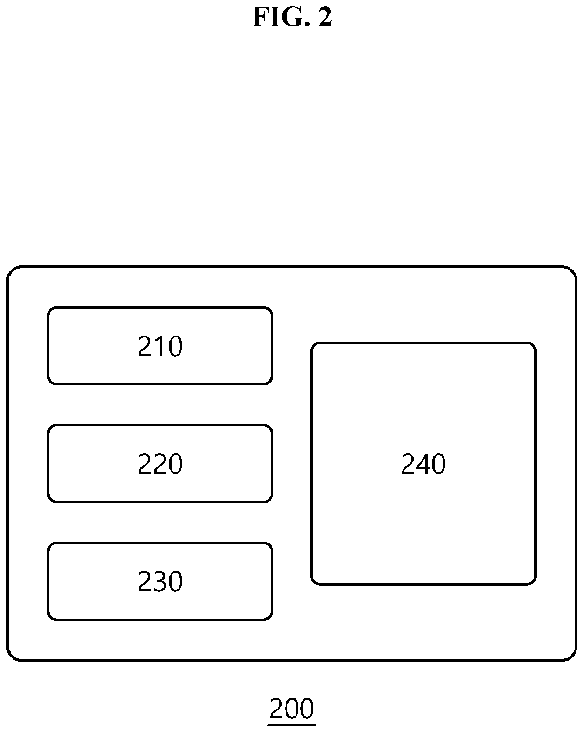

FIG. 2 is a block diagram for explaining an internal configuration of a computing device (200) in accordance with an embodiment of the present specification. The computing device (200) can be applied to one or more user devices (110-1, 110-2) or servers (120-140) described above with reference to FIG. 1, and each device and server may have the same or similar internal configuration by adding or excluding some components.

Referring to FIG. 2, the computing device (200) may include a memory (210), a processor (220), a communication module (230), and a transceiver unit (240). The memory (210) is a non-transitory computer-readable storage medium and may include a permanent mass storage device such as Random Access Memory (RAM), Read Only Memory (ROM), a disk drive, a Solid State Drive (SSD), flash memory, and the like. Here, a permanent mass storage device such as ROM, SSD, flash memory, disk drive, and the like may be included as a separate permanent storage device distinct from the memory (210) in the device or server described above. Additionally, the memory (210) may store an operating system and at least one program code (for example, code for a browser installed and executed on a user device (110) or an application installed on a user device (110) for provision of a specific service). These software components may be loaded from a computer-readable recording medium separate from the memory (210). The separate computer-readable recording medium may include computer-readable recording media such as a floppy drive, disk, tape, DVD/CD-ROM drive, memory card, and the like.

In another embodiment, software components may be loaded into the memory (210) via the communication module (230) rather than a computer-readable recording medium. For example, at least one program may be loaded into the memory (210) based on a computer program (for example, the application described above) installed by files provided by developers or a file distribution system distributing installation files of the application (for example, the server described above) through the network (1).

The processor (220) can be configured to process instructions of a computer program by performing basic arithmetic, logic, and input/output operations. Instructions may be provided to the processor (220) by the memory (210) or the communication module (230). For example, the processor (220) can be configured to execute instructions received according to program code stored in a recording device such as the memory (210).

The communication module (230) can provide a function for communication between the user device (110) and the servers (120-140) via the network (1), and can provide a function for each of the device (110) and/or the servers (120-140) to communicate with other electronic devices.

The transceiver unit (240) may be a means for interfacing with an external input/output device (not shown). For example, an external input device may include devices such as a keyboard, mouse, microphone, camera, and the like, and an external output device may include devices such as a display, speaker, haptic feedback device, and the like. As another example, the transceiver unit (240) may be a means for interfacing with a device integrated with functions for both input and output, such as a touchscreen.

Additionally, in other embodiments, the computing device (200) may include more components than the components in FIG. 2 depending on the nature of the device to which it is applied. For example, when the computing device (200) is applied to a user device (110), it may be implemented to include at least some of the input/output devices described above, or may further include other components such as a transceiver, a Global Positioning System (GPS) module, a camera, various sensors, a database, and the like. As a more specific example, when the user device is a smartphone, it may be implemented to further include various components generally included in a smartphone, such as an acceleration sensor, a gyroscope sensor, a camera module, various physical buttons, a button using a touchpad, an input/output port, a vibrator for vibration, and the like.

In an embodiment, the Smart All-in-One Cardiac Patch System described hereinafter can operate through the computing device (200) of FIG. 2 based on the network of FIG. 1. More specifically, the Smart All-in-One Cardiac Patch System may include a Smart All-in-One Cardiac Patch acquiring cardiac-related signals, a user device acquiring signals measured through the cardiac patch, and a Smart All-in-One Cardiac Patch Server acquiring and processing signals measured from a plurality of user devices. Here, each device may be a device operating based on the computing device (200) of FIG. 2, but is not limited to the embodiment. Additionally, each device can exchange signals based on the network of FIG. 1, but is not limited to the embodiment. Hereinafter, for convenience of description, the description is based on the Smart All-in-One Cardiac Patch, the user device, and the Smart All-in-One Cardiac Patch Server, but may not be limited to the names.

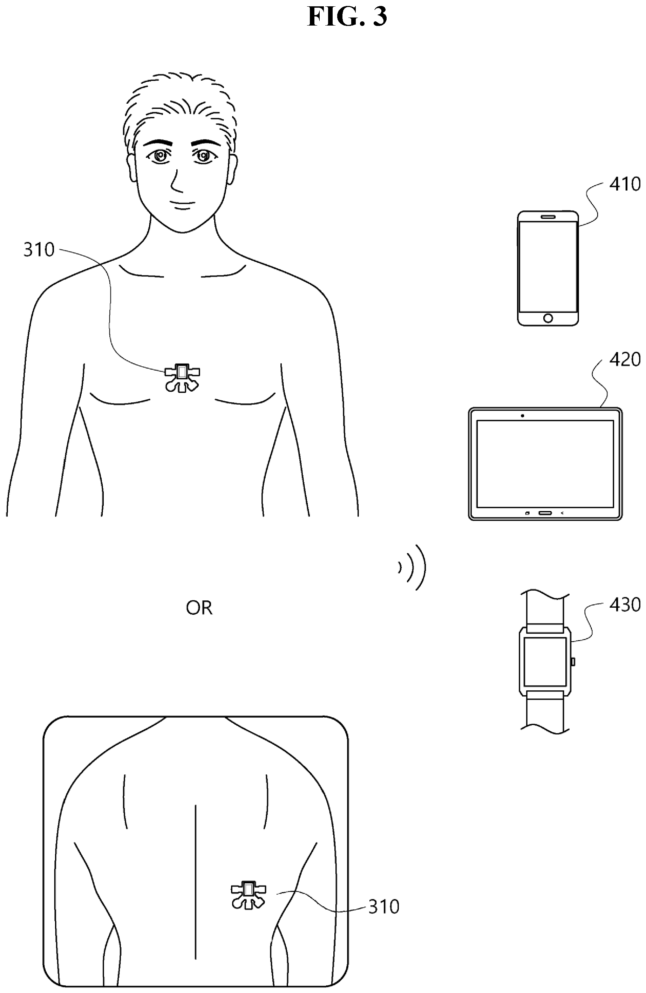

FIG. 3 is a diagram showing a Smart All-in-One Cardiac Patch Device and a plurality of user devices in accordance with an embodiment of the present specification. Referring to FIG. 3, the Smart All-in-One Cardiac Patch (310) can be attached to the human body. The Smart All-in-One Cardiac Patch (310) can acquire cardiac-related signals while being attached to the human body. Here, the cardiac-related signals acquired by the Smart All-in-One Cardiac Patch (310) can include at least one of Electrocardiogram (ECG), Seismocardiogram (SCG), and Phonocardiogram (PCG). Here, ECG may be an electrical signal related to cardiac beating, SCG may be a low-frequency vibration signal of the heart, and PCG may be a cardiac acoustic signal. That is, the Smart All-in-One Cardiac Patch (310) can acquire at least one or more of electrical signals, vibration signals, and acoustic signals related to cardiac beating.

Here, in an embodiment, the Smart All-in-One Cardiac Patch (310) can be attached at the heart location on the front of the human body to acquire cardiac-related signals. As another embodiment, the Smart All-in-One Cardiac Patch (310) can be attached at the heart location on the back of the human body (dorsal region) to acquire cardiac-related signals. As yet another embodiment, a plurality of Smart All-in-One Cardiac Patches (310) can be attached to the human body. Specifically, the Smart All-in-One Cardiac Patch (310) can be attached to both the heart location on the front and the back of the human body. Here, cardiac-related signals can be obtained from each of the plurality of Smart All-in-One Cardiac Patches (310) and compared, and since cardiac-related signals are acquired based on the compared signals, the measurement accuracy of cardiac-related signals can be improved. As another embodiment, the Smart All-in-One Cardiac Patch (310) can further acquire additional information related to cardiac beating or the human body. In an embodiment, the Smart All-in-One Cardiac Patch (310) can further acquire information regarding the user's body temperature or moisture content of the epidermis. The user's body temperature or moisture content of the epidermis may be information that can affect the case of acquiring cardiac-related signals through the Smart All-in-One Cardiac Patch (310), and the Smart All-in-One Cardiac Patch (310) can further acquire such information. As a specific embodiment, in a case of low temperature such as winter, if the user's body temperature is lowered, cardiac beating may slow down, and the Smart All-in-One Cardiac Patch (310) can further acquire body temperature, external temperature, and other information.

As another embodiment, the Smart All-in-One Cardiac Patch (310) can further acquire location information, environmental information, and other information. Specifically, the cardiac signal pattern of a user using the Smart All-in-One Cardiac Patch (310) may be different in a case of being indoors or in a case of being outdoors. Alternatively, cardiac-related signals may be different between a user's home where psychological stability is felt and an office space where work is performed. Here, the Smart All-in-One Cardiac Patch (310) can jointly measure the user's location information and transmit it to the user device. As another embodiment, the Smart All-in-One Cardiac Patch (310) can acquire ambient information as environmental information. In an embodiment, environmental information may be information affecting cardiac beating, such as temperature, humidity, air flow, and other information surrounding the user. That is, the Smart All-in-One Cardiac Patch (310) can further acquire information related to cardiac beating, but is not limited thereto.

Thereafter, the Smart All-in-One Cardiac Patch (310) can transmit the aforementioned acquired signal to the user devices (410, 420, 430). In an embodiment, the user devices (410, 420, 430) are devices used by a user who has attached the Smart All-in-One Cardiac Patch (310), and may be a smartphone (410), a tablet (420), a smartwatch (430), and other devices, and is not limited to a specific type. However, hereinafter, for convenience of description, the description is based on the tablet (420) among the user devices, but may not be limited thereto. Thereafter, the user devices (410, 420, 430) can perform monitoring based on signals acquired from the Smart All-in-One Cardiac Patch (310). Additionally, the user devices (410, 420, 430) can transmit signals acquired from the Smart All-in-One Cardiac Patch (310) to the server. As another embodiment, a user device may be a device operating within a preset distance from the Smart All-in-One Cardiac Patch (310). As a specific embodiment, the user device may be a smartwatch (430). Here, the user device can acquire the aforementioned location information and environmental information within a preset distance from the Smart All-in-One Cardiac Patch (310), and is not limited to the aforementioned embodiment. In an embodiment, the smartwatch (430) among the user devices can measure the aforementioned location information and environmental information within a preset distance from the Smart All-in-One Cardiac Patch (310) and transmit them to another user device (e.g., tablet 420). That is, the tablet (420) can acquire cardiac-related signals from the Smart All-in-One Cardiac Patch (310) and can further acquire additional user-related information through the smartwatch (430), but may not be limited to the embodiment.

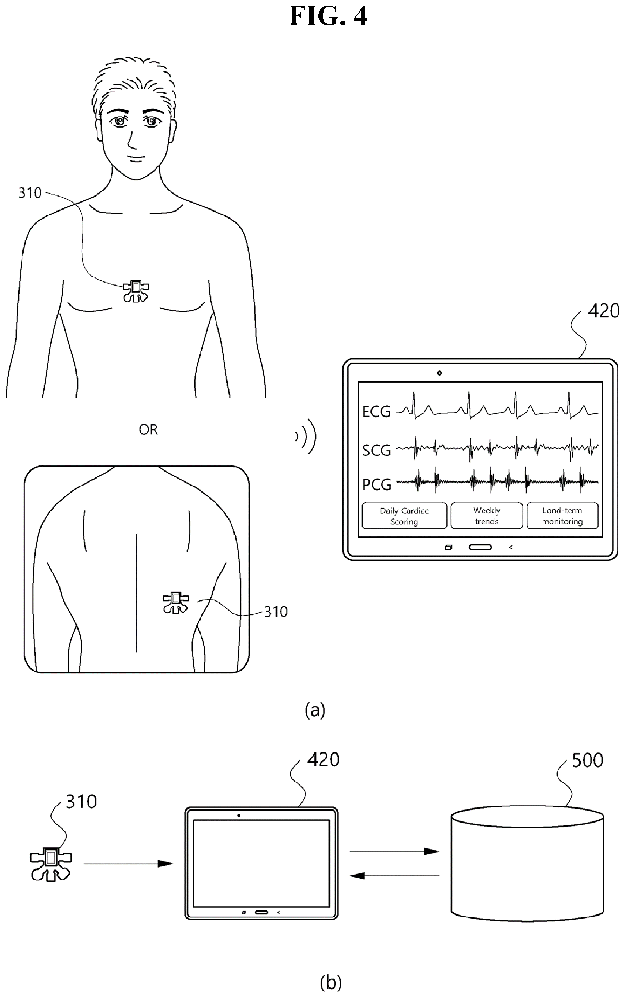

FIG. 4 is a diagram showing a Smart All-in-One Cardiac Patch System in accordance with an embodiment of the present specification. Referring to FIG. 4, the Smart All-in-One Cardiac Patch System may include a Smart All-in-One Cardiac Patch (310), a user device (420), and a cloud (or server, 500). Additionally, the Smart All-in-One Cardiac Patch System may further include other components, and may not be limited to a specific form. Referring to FIG. 4(a), the user device (420) can acquire the aforementioned signal from the Smart All-in-One Cardiac Patch (310). In an embodiment, the signal can further include ECG, SCG, PCG, and other information, and is not limited to a specific embodiment. Here, in an embodiment, the Smart All-in-One Cardiac Patch (310) can be attached at the heart location on the front of the human body to acquire cardiac-related signals. As another embodiment, the Smart All-in-One Cardiac Patch (310) can be attached at the heart location on the back of the human body (dorsal region) to acquire cardiac-related signals, which is as described above.

As yet another embodiment, a plurality of Smart All-in-One Cardiac Patches (310) can be attached to the human body. Specifically, the Smart All-in-One Cardiac Patch (310) can be attached to both the heart location on the front and the back of the human body. Here, cardiac-related signals can be obtained from each of the plurality of Smart All-in-One Cardiac Patches (310) and compared, and since cardiac-related signals are acquired based on the compared signals, the measurement accuracy of cardiac-related signals can be improved.

Additionally, the user device (420) can further acquire additional information. Hereinafter, for convenience of description, signals and additional information acquired by the user device (420) from the Smart All-in-One Cardiac Patch (310) are referred to as cardiac-related information, but may not be limited thereto. When the user device (420) acquires cardiac-related information from the Smart All-in-One Cardiac Patch (310), the user device (420) can display and provide the cardiac-related information to the user. Additionally, in an embodiment, the user device (420) can acquire cardiac-related information from the Smart All-in-One Cardiac Patch (310) in real-time or based on a preset period. The user device (420) can analyze and monitor at least one of user condition information and disease information through cardiac-related information acquired in real-time or based on a preset period. Here, the user device (420) can display and provide analysis information to the user. In an embodiment, the user device (420) can provide analysis information based on a preset period. As a specific embodiment, the user device (420) can provide at least one of daily information, weekly information, monthly information, and long-term based information, and is not limited to a specific form. That is, the user device (420) can acquire cardiac-related information from the Smart All-in-One Cardiac Patch (310) in real-time or based on a preset period, derive analysis information from the cardiac-related information, and provide it to the user.

As another embodiment, referring to FIG. 4(b), the user device (420) can transmit cardiac-related information acquired from the Smart All-in-One Cardiac Patch (310) to the cloud (500). Here, the cloud (500) can acquire signals from each of a plurality of user devices (420) that acquire signals from the Smart All-in-One Cardiac Patch (310), perform analysis, and transmit the analyzed information to the user device (420). As another embodiment, the cloud (500) can perform training on a condition monitoring learning model based on cardiac-related information acquired from a plurality of user devices (420). Thereafter, the cloud (500) can transmit the trained condition monitoring learning model to each user device (420). Each user device (420) can perform inference by providing cardiac-related information acquired from the user as input to the condition monitoring learning model, and can automatically derive whether an abnormal condition exists in the user as an output value, which will be described below.

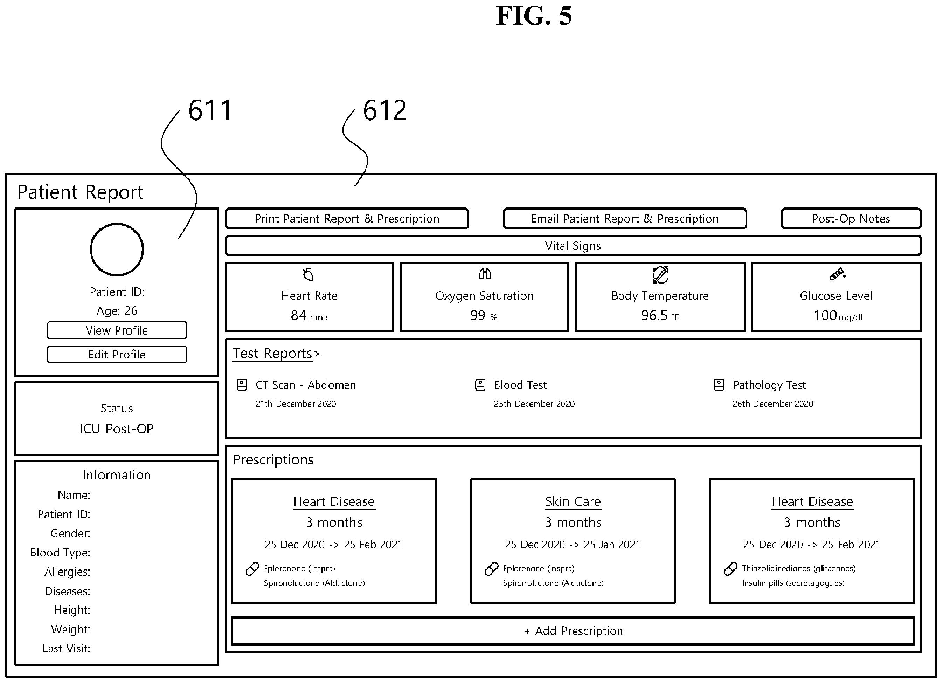

Additionally, in an embodiment, FIG. 5 is a diagram showing a method by which the user device (420) provides analysis information in accordance with an embodiment of the present specification. Referring to FIG. 5, the user device (420) can acquire signals from the Smart All-in-One Cardiac Patch (310) in real-time or based on a preset period as described above, and can perform analysis thereon. Thereafter, the user device (420) can generate and display the user report information of FIG. 5. In an embodiment, an interface providing the user report information can include user-related information (611) and analysis information (612). In an embodiment, the user-related information (611) can include the user's name, age, gender, height, weight, blood type, allergies, diseases, and other information about the user, and is not limited to a specific form. Additionally, the user report information can further include analysis information (612) derived based on cardiac-related information. In an embodiment, the analysis information (612) can include heart rate, oxygen saturation, body temperature, glucose level, and other information. Additionally, the analysis information (612) can include test information performed based on cardiac-related information acquired from the Smart All-in-One Cardiac Patch (310). Here, the test information may be information provided by analyzing the acquired cardiac-related information according to a specific purpose. As another embodiment, the analysis information (612) can further include prescription information. The user device can analyze cardiac-related information acquired from the Smart All-in-One Cardiac Patch (310) based on the prescription information, and can generate and display corresponding information. That is, the user device (420) can derive user report information based on cardiac-related information acquired from the Smart All-in-One Cardiac Patch (310), and can display the information. Through the above, the user can recognize their own condition, and can perform additional actions based on the test information and prescription information.

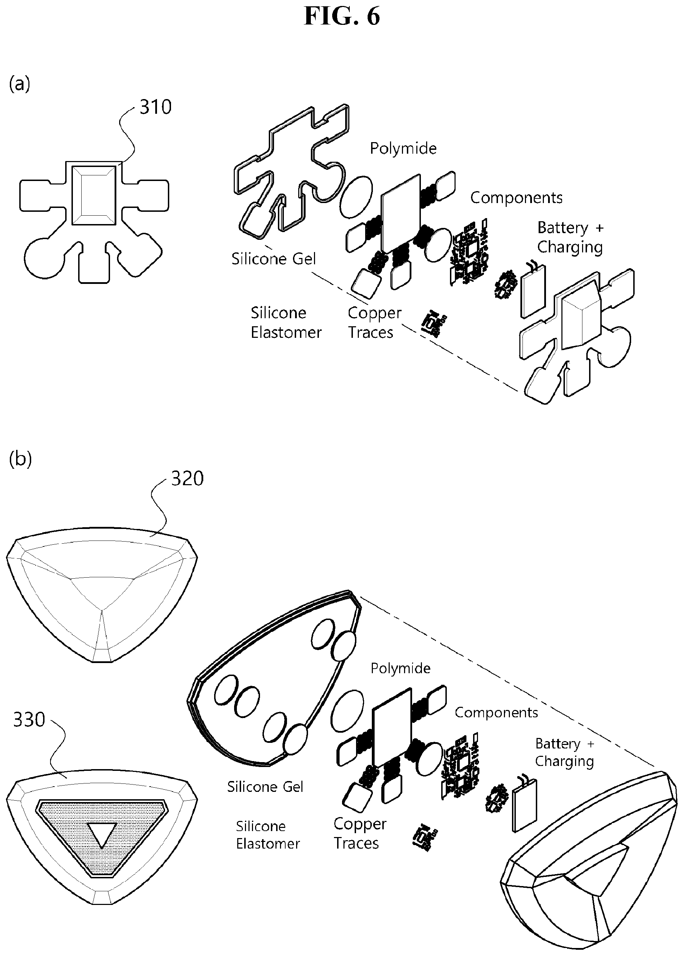

FIG. 6 is a diagram showing the structure of a Smart All-in-One Cardiac Patch in accordance with an embodiment of the present specification. Referring to FIG. 6, the Smart All-in-One Cardiac Patch may be either a first type (310) or a second type (320). Additionally, the Smart All-in-One Cardiac Patch may be of another type or form, and is not limited to a specific form. In an embodiment, referring to FIG. 6(a), the Smart All-in-One Cardiac Patch can include silicone gel and silicone elastomer. The silicone gel is a deformable material that can allow the Smart All-in-One Cardiac Patch to be attached according to the shape of the human body surface so as not to detach. The silicone elastomer may be a material with excellent elasticity, absorption, and tensile strength, and can allow the Smart All-in-One Cardiac Patch to be attached to and maintained on the human body. That is, the part attached to the human body surface may be composed of silicone gel and silicone elastomer, and through this, the Smart All-in-One Cardiac Patch can be maintained in a state of being attached to the human body. Additionally, electrodes and a wireless charging coil may be included inside the silicone gel and silicone elastomer. Here, the electrodes may be ECG electrodes, and may be gold electrodes composed of gold, but are not limited thereto, and electrodes of other materials may be configured. Additionally, the wireless charging coil may be a component that performs battery charging, and based on this, the Smart All-in-One Cardiac Patch can be charged. Additionally, polyimide and copper trace may be included inside the silicone gel and silicone elastomer. Here, the polyimide may be a polymer material having thermal stability and high mechanical strength. In an embodiment, the first type (310) Smart All-in-One Cardiac Patch may be identically configured in a polyimide form. Through this, when the Smart All-in-One Cardiac Patch is attached to the human body, the polyimide can deform together when the Smart All-in-One Cardiac Patch stretches or bends, and can allow the Smart All-in-One Cardiac Patch to maintain attachment without detaching from the human body.

Additionally, the copper trace can allow signals acquired by the Smart All-in-One Cardiac Patch to be traced and transmitted through the high conductivity of copper. Additionally, the Smart All-in-One Cardiac Patch can further include components for acquiring signals and transmitting them to the user device, and a battery for operating the Smart All-in-One Cardiac Patch. Here, in an embodiment, the components may include configurations for acquiring ECG as an electrical signal, SCG as a low-frequency vibration signal, and PCG as an acoustic signal as described above, and are not limited to a specific form. Additionally, in an embodiment, the battery can perform a role of maintaining power to transmit signals acquired by the Smart All-in-One Cardiac Patch to the user device. In an embodiment, the Smart All-in-One Cardiac Patch is a low-power device that can maintain power for a long period through the battery. As another embodiment, the battery may be of a form that is attached to the human body and charged according to the user's movement. As described above, since the Smart All-in-One Cardiac Patch is a low-power device, it can operate through power that is attached to the human body and charged according to the user's movement, but may not be limited to the embodiment.

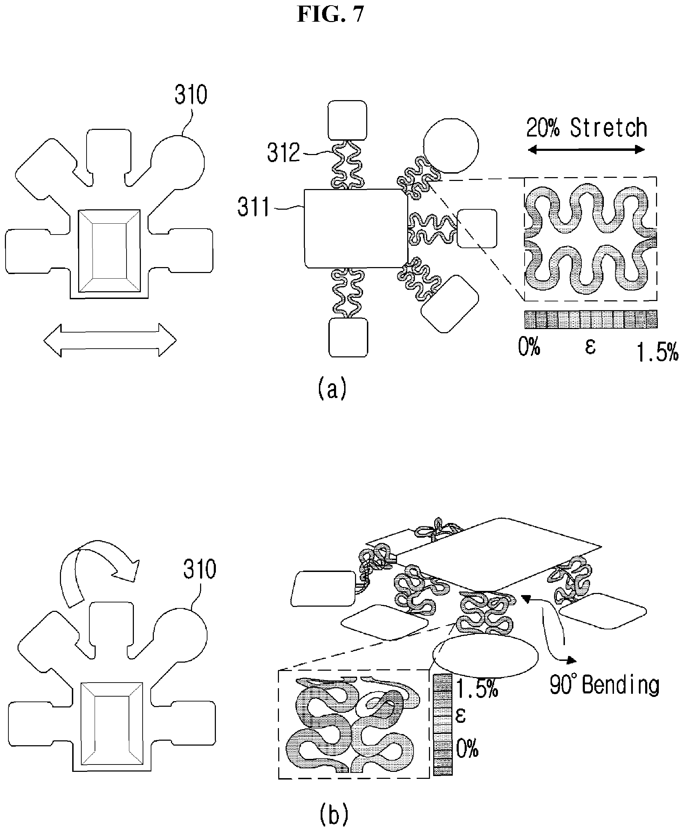

FIG. 7 is a diagram showing the internal structure of a Smart All-in-One Cardiac Patch in accordance with an embodiment of the present specification. Referring to FIG. 7, the Smart All-in-One Cardiac Patch can include the aforementioned polyimide. In an embodiment, FIG. 7 is described based on the first type Smart All-in-One Cardiac Patch (310) for convenience of description, but may not be limited thereto. Referring to FIG. 7, the polyimide in the Smart All-in-One Cardiac Patch (310) can include a configuration part (311) and a connecting part (312). Here, the configuration part (311) may be an area within the polyimide where the aforementioned components are attached and located. In an embodiment, in FIG. 7(a) and FIG. 7(b), the configuration part (311) may be composed of a first configuration part (311-1) in the central area and a second configuration part (311-2) in the distal area, but may not be limited to the embodiment. Additionally, each configuration part (311) can be mutually connected through the connecting part (312). Here, the aforementioned components may not be attached to the connecting part (312). In an embodiment, the connecting part (312) can deform according to the relaxation or contraction of the Smart All-in-One Cardiac Patch (310). More specifically, the Smart All-in-One Cardiac Patch (310) may be composed of silicone gel as described above, and the silicone gel can relax or contract. In an embodiment, the connecting part (312) can relax or contract together according to the relaxation or contraction of the silicone gel. As a specific embodiment, in FIG. 7(a), the connecting part (312) can relax up to a preset relaxation rate so that the Smart All-in-One Cardiac Patch (310) can relax. In an embodiment, the relaxation rate may be 20%, but this is merely an example and is not limited to the embodiment.

Additionally, in an embodiment, the connecting part (312) can deform according to bending. More specifically, the Smart All-in-One Cardiac Patch (310) may be composed of silicone gel as described above, and the silicone gel can bend. In an embodiment, the connecting part (312) can bend together according to the bending of the silicone gel. As a specific embodiment, in FIG. 7(b), the connecting part (312) can bend up to a preset angle. Here, the preset bending angle may be 90 degrees, but this is merely an example and is not limited to the embodiment. The Smart All-in-One Cardiac Patch (310) can be attached to the human body based on the aforementioned structure. In an embodiment, the Smart All-in-One Cardiac Patch (310) can relax or contract according to the location at which it is attached, and a portion thereof can bend. That is, the Smart All-in-One Cardiac Patch (310) can be partially deformed in shape according to the human body location at which it is attached, and through this, the adhesion strength to the human body can be improved. Additionally, the first configuration part (311-1) in the central area, as the configuration part (311), can include components that receive cardiac-related signals measured from the human body and transmit the signals to the user device as an external device. Additionally, the second configuration part (311-2) in the distal area can include sensors for directly measuring cardiac-related signals from the human body, and the signals acquired from the sensors can be transmitted to the components of the first configuration part (311-1), and through the above, the Smart All-in-One Cardiac Patch (310) can acquire and transmit cardiac-related signals.

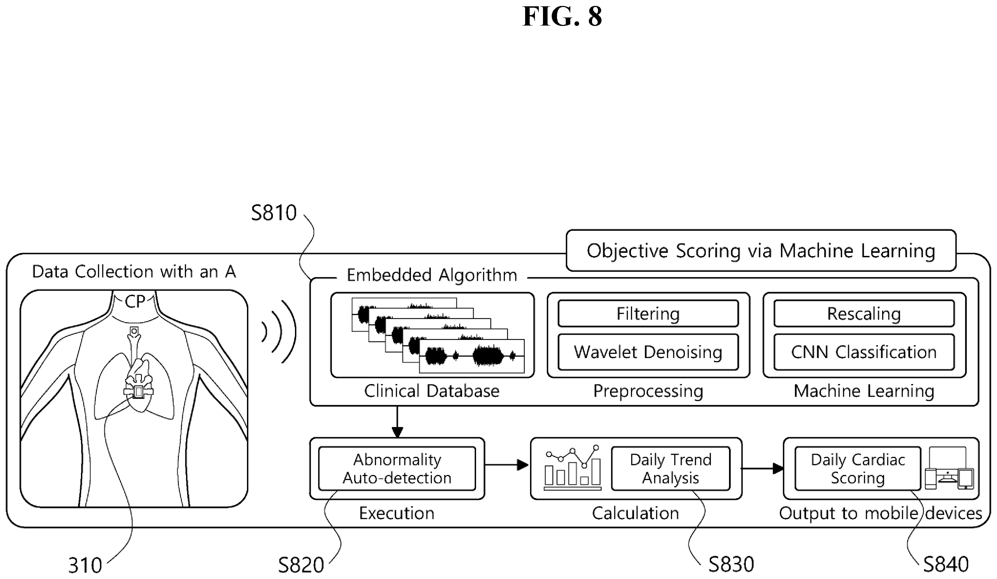

FIG. 8 is a diagram showing a method of detecting whether an abnormality exists based on machine learning in accordance with an embodiment of the present specification. Referring to FIG. 8, the Smart All-in-One Cardiac Patch System can provide cardiac analysis information based on Artificial Intelligence/Machine Learning (AI/ML). More specifically, the Smart All-in-One Cardiac Patch (310) can acquire cardiac-related signals and transmit them to a user device, which is as described above. Here, the user device can perform inference based on signals acquired by an embedded algorithm or a condition monitoring learning model (S810) to automatically detect whether an abnormality exists (S820). In an embodiment, the user device can be equipped with an embedded algorithm or a condition monitoring learning model. Here, the embedded algorithm or condition monitoring learning model can automatically detect abnormal conditions in the user. As a specific embodiment, the user device can acquire cardiac-related signals from the Smart All-in-One Cardiac Patch (310) and compare them with information stored in a database. Additionally, the user device can perform filtering, wavelet denoising, and other preprocessing operations before performing inference based on the preprocessed information. In an embodiment, the learning model can perform inference based on rescaling or Convolutional Neural Network (CNN) classification operation, whereby whether the acquired signal is abnormal can be automatically detected. Thereafter, the user device can calculate analysis information based on information regarding whether an abnormality exists (S830) and can display the analysis information on the user device (S840). In an embodiment, the user device can perform daily trend analysis to compare signal information acquired on a daily basis with information regarding whether an abnormality exists, and can derive scoring information based thereon and display it on the user device. That is, the user device can analyze signals acquired from the Smart All-in-One Cardiac Patch (310) based on AI/ML to provide analysis information to the user, and is not limited to a specific form. Here, in an embodiment, the user device can be provided with a pre-trained condition monitoring learning model on a cloud basis, and can provide cardiac-related information as input to the condition monitoring learning model to derive whether an abnormality exists as an output value.

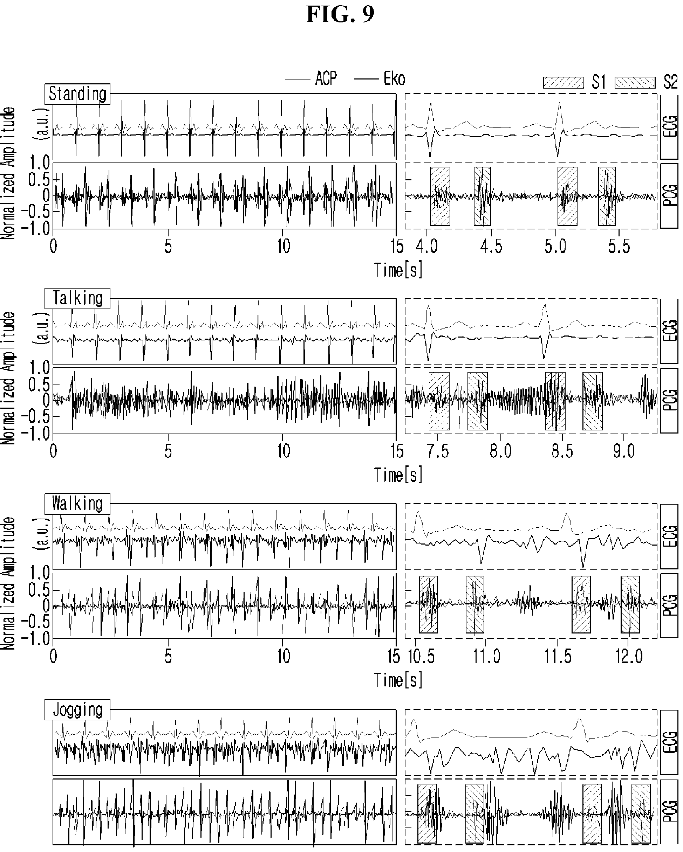

Additionally, in an embodiment, signals acquired from the Smart All-in-One Cardiac Patch (310) can be analyzed by reflecting user condition information. In an embodiment, FIG. 9 is a diagram showing a method of analyzing signals obtained based on user condition information in accordance with an embodiment of the present specification. Referring to FIG. 9, there is a need for signals acquired from the Smart All-in-One Cardiac Patch (310) to be processed by reflecting user condition information. Specifically, the user condition can include a standing state, a talking state, a walking state, a jogging state, and other states. Here, cardiac-related signals can be acquired differently depending on the user condition. In an embodiment, when a user is in conversation with another user (that is, in a talking state), a user voice signal can be included as noise in the cardiac-related signal. Therefore, there is a need to derive information in the form of removing the corresponding noise from the cardiac-related signal. As another embodiment, when the user is jogging (that is, in a jogging state), the number of cardiac beats can increase, and accordingly, the acquired cardiac-related signals can be different. In consideration of the aforementioned points, the user device can further acquire user condition information when acquiring cardiac-related signals. Additionally, user condition information can be reflected in the aforementioned embedded algorithm or learning model. Detection of whether an abnormality exists can be derived based on user condition information. As a specific embodiment, when the user is jogging, the embedded algorithm or learning model can perform inference based on the jogging state as user condition information and can determine whether an abnormality exists. That is, the embedded algorithm or learning model can build normal information of the jogging state for the user, and if signals acquired in the jogging state are different from the normal information, it can automatically detect whether an abnormality exists. However, this is merely one example and may not be limited to the embodiment. That is, signals acquired from the Smart All-in-One Cardiac Patch (310) can be analyzed by reflecting the user's condition, and the analyzed information can be displayed through the user device.

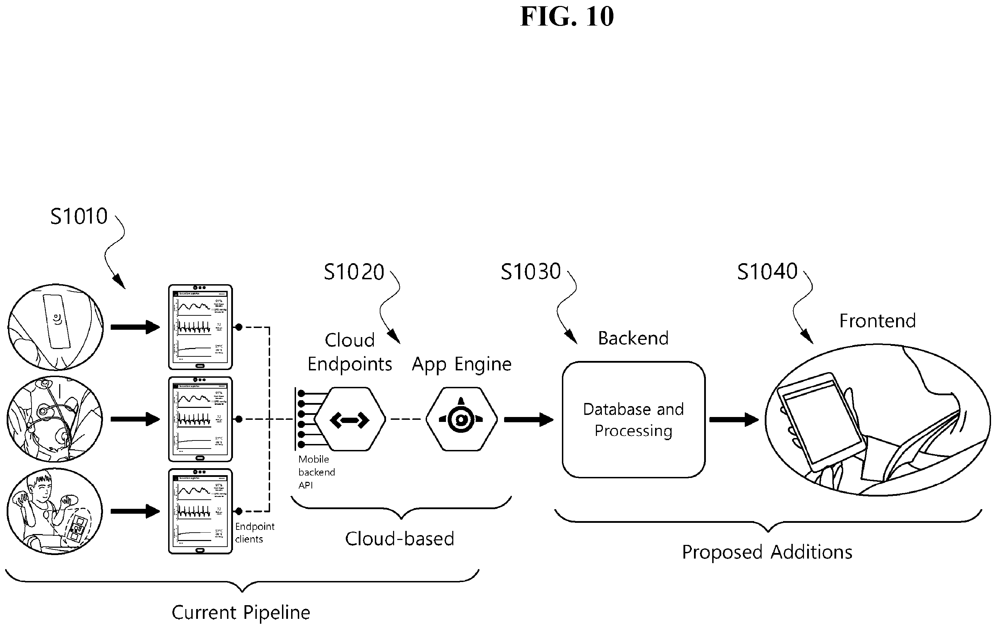

As another embodiment, the embedded algorithm or learning model that detects whether an abnormality exists in cardiac-related signals can be trained based on a cloud, and the user device can acquire the embedded algorithm or learning model trained based on the cloud. FIG. 10 is a diagram showing a method of training a learning model on a cloud basis in accordance with an embodiment of the present specification. Referring to FIG. 10, the condition monitoring learning model that detects whether an abnormality exists in cardiac-related signals in the Smart All-in-One Cardiac Patch System can be trained on a cloud basis. In an embodiment, a plurality of user devices can be linked to a cloud endpoint, and each of the plurality of user devices can be linked with a Smart All-in-One Cardiac Patch of a specific user to acquire cardiac-related information (S1010). Each of the plurality of user devices can transmit the acquired cardiac-related information to the cloud (S1020), and the cloud can transmit the corresponding information to the backend based on an app engine (S1030). Here, the backend can use the cardiac-related information acquired from the plurality of user devices through the cloud for training of the condition monitoring learning model. In an embodiment, the backend can update the condition monitoring learning model based on the acquired plurality of signals and a database, and can transmit the updated learning model back to the user device (S1040). The user device can perform inference using cardiac-related signals acquired from the Smart All-in-One Cardiac Patch as input based on the acquired condition monitoring learning model to detect whether an abnormality exists.

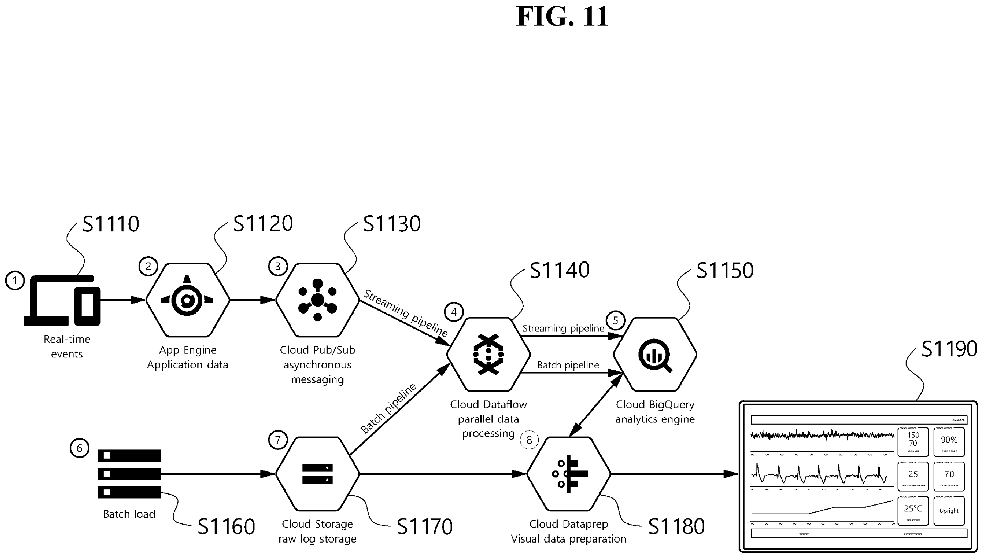

In an embodiment, FIG. 11 is a diagram showing a method of updating a condition monitoring learning model in accordance with an embodiment of the present specification. Referring to FIG. 11, the cloud can acquire real-time information on a streaming basis. That is, cardiac-related information is acquired in real-time from each Smart All-in-One Cardiac Patch linked with a plurality of user devices (S1110), and the cardiac-related information can be generated as application data (S1120) and transmitted to the cloud (S1130). Additionally, the cloud can acquire cardiac-related information for the plurality of user devices and perform data processing (S1140). Here, in an embodiment, batch load information can be further acquired (S1110), and the acquired batch load information can be transmitted to the cloud (S1170). Here, the batch load information can be information related to a data set on which training is to be performed. In an embodiment, the cloud can perform data processing in parallel on the cardiac-related information acquired in real-time and the batch load information (S1140), and can perform analysis based on each piece of information performed in parallel to update the condition monitoring learning model (S1150). Thereafter, the updated information can be transmitted to the user device (S1180), and the user device can display the analyzed cardiac-related information based on the updated condition monitoring learning model (S1190). Additionally, the batch load information can also be transmitted to the user device, and the user device can analyze and display cardiac-related signals based on the corresponding information.

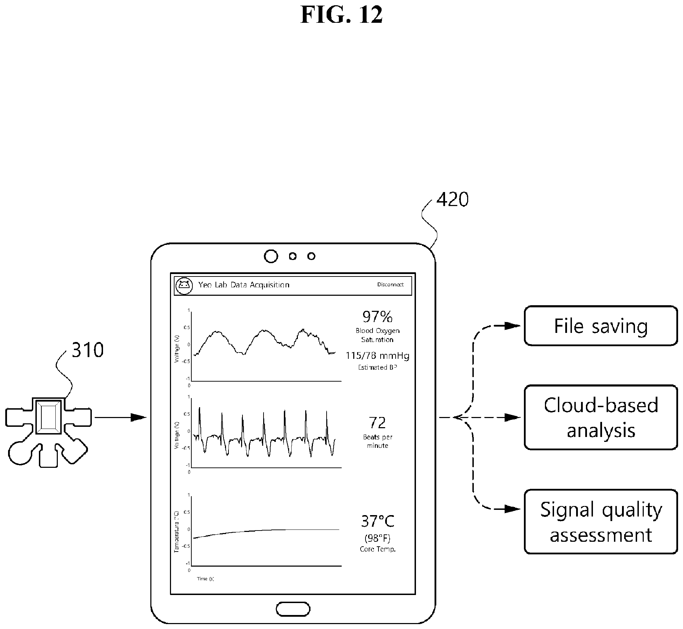

In an embodiment, FIG. 12 is a diagram showing a method of displaying cardiac-related information by a user device in accordance with an embodiment of the present specification. Referring to FIG. 12, the user device (420) can derive and display analysis information based on signals acquired from the Smart All-in-One Cardiac Patch, whereby information can be provided to the user. Here, the user device (420) can perform analysis on cardiac-related signals acquired based on a condition monitoring learning model trained on a cloud basis or an embedded algorithm. Additionally, the user device (420) can store signals acquired through the Smart All-in-One Cardiac Patch (310), and perform monitoring based on the stored signal information to analyze the user condition. Through this, the user device (420) can provide analysis information to the user, and the analysis information can be provided at preset intervals. Additionally, the analysis information can be provided by reflecting user condition information, which is as described above.

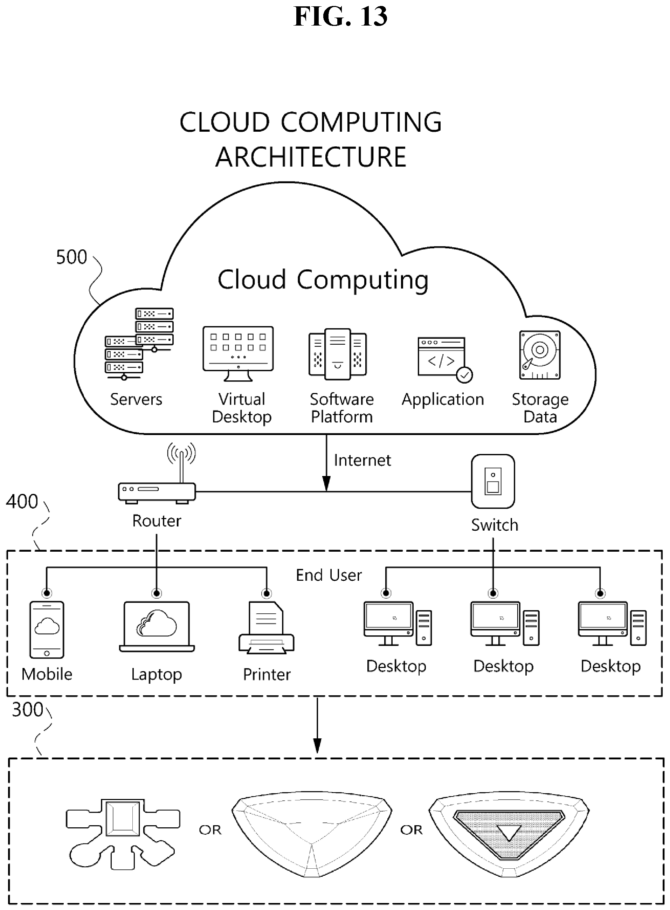

In an embodiment, FIG. 13 is a diagram showing a Smart All-in-One Cardiac Patch System in accordance with an embodiment of the present specification. In an embodiment, the Smart All-in-One Cardiac Patch System can include, as described above, a Smart All-in-One Cardiac Patch (300), a user device (400), and a cloud (500). In an embodiment, the Smart All-in-One Cardiac Patch (300) can be configured in different types, as described above. Specifically, the Smart All-in-One Cardiac Patch (300) can be a first type (310), such as a first-1 type (310-1) or a first-2 type (310-2), but may not be limited to a specific form. Additionally, it can be a second type (320), such as a second-1 type (320-1) or a second-2 type (320-2), but may not be limited to a specific form. Additionally, in an embodiment, the Smart All-in-One Cardiac Patch can be manufactured as other types, and is not limited to a specific form. Here, the Smart All-in-One Cardiac Patch (300) can be linked with each user device (400), and the user device (400) can be a smartphone, a laptop, a desktop, and other devices. Thereafter, the cloud (500) can receive cardiac-related information acquired from each user device (400). In an embodiment, each of the user devices (400) can transmit the acquired cardiac-related information to the cloud (500) through a network. Here, the cloud (500) can be a server, a platform, stored data, and other forms, and may not be limited to a specific form.

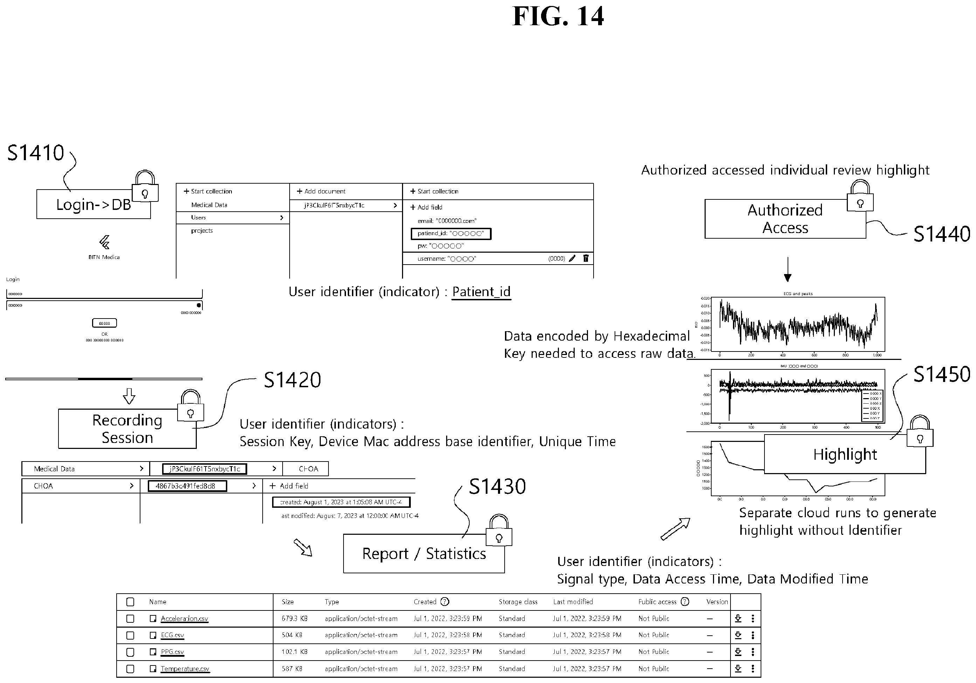

In an embodiment, FIG. 14 is a diagram showing a method of performing a user information security operation in accordance with an embodiment of the present specification. In an embodiment, the Smart All-in-One Cardiac Patch System can acquire cardiac-related information from a user device to which each Smart All-in-One Cardiac Patch is connected. However, the information acquired from each Smart All-in-One Cardiac Patch can be personal information for each user, and a security operation for the corresponding information can be necessary for user privacy and security. Referring to FIG. 14, the user device allocates user identification information for the user, and can acquire cardiac-related signals from the Smart All-in-One Cardiac Patch based on the user identification information (S1410). Additionally, the user device can further acquire cardiac-related additional information based on the user identification information to generate cardiac-related information, which is as described above. In an embodiment, the user identification information can be a user ID (patient_id), and the Smart All-in-One Cardiac Patch can sense and transmit cardiac-related signals corresponding to the corresponding user identification information. Thereafter, the cardiac-related information including the acquired cardiac-related signals and additional information can be recorded according to a session key, a device MAC address-based identifier, and a unique time based on the user identification information (S1420). The user device can perform analysis based on the information recorded based on the user identification information to generate report information to be reported to the cloud (S1430). That is, the user device can acquire cardiac-related information for the corresponding user based on the user identification information, generate report information based thereon, and transmit the corresponding report information to the cloud. Through the above, the cloud can acquire cardiac-related information for each user. Here, the report information reported from the user device can be transmitted to the cloud together with a signal type, a data access time, and a data modification time based on the user identification information. Here, the report information transmitted based on the user identification information can be information identified per user, and when the information identified per user is used as is, privacy and security problems can occur. Therefore, the cloud can perform an operation of deriving record information (highlight) with the identifier excluded from the acquired report information. That is, the cloud can acquire report information, signal type, data access time, and data modification time information from the user device based on the user identification information, and then generate record information with the user identification information excluded. Thereafter, the cloud can grant authorized administrator access (S1440). Here, the record information can be encoded through a hexadecimal key capable of accessing raw data, and the cloud can update the learning model or embedded algorithm based on the encoded data (S1450).

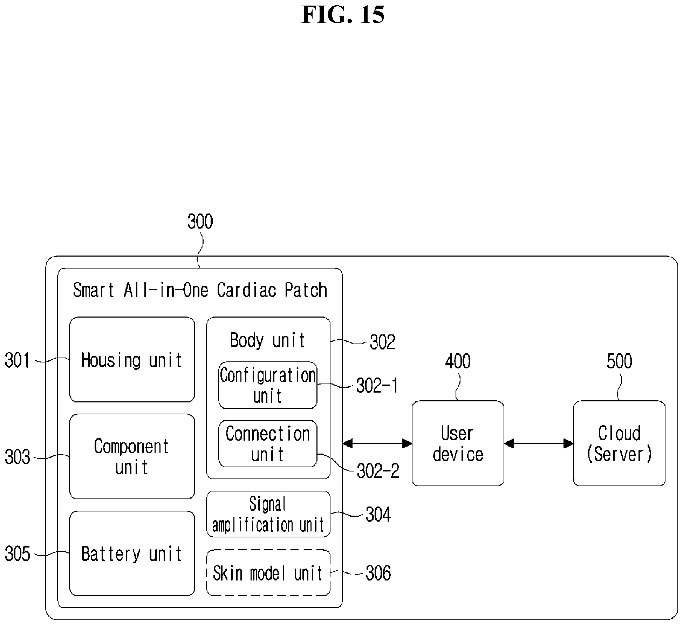

FIG. 15 is a diagram showing a Smart All-in-One Cardiac Patch method and device in accordance with an embodiment of the present specification. Referring to FIG. 15, the Smart All-in-One Cardiac Patch (300) can include at least one or more of a housing unit (301), a body unit (302), a component unit (303), a signal amplification unit (304), a battery unit (305), and a skin model unit (306). In an embodiment, the Smart All-in-One Cardiac Patch (300) can be of any one form among the aforementioned first type (310) or second type (320) and other types, but may not be limited thereto. Here, the housing unit (301) can include the aforementioned silicone gel and silicone elastomer. That is, the housing unit (301) can protect each component from the outside of the body unit (302), component unit (303), signal amplification unit (304), and battery unit (305). However, the skin model unit (306) can be a configuration that enables the Smart All-in-One Cardiac Patch (300) to be attached and maintained to the human body on the outside of the housing unit (301). Additionally, electrodes and a wireless charging coil can be positioned inside the housing unit (301). Here, the electrode may be an ECG electrode, and the ECG electrode can be composed of gold material, but is not limited to the embodiment. In an embodiment, the outside of the housing unit (301) can be attached to the human body, and cardiac-related signals generated from the human body can be transmitted through the ECG electrode. Additionally, the wireless charging coil can be a configuration that charges the battery of the Smart All-in-One Cardiac Patch, which is as described above. Inside the housing unit (301), the body unit (302) can include a configuration unit (302-1) and a connection unit (302-2). In an embodiment, the body unit (302) can include a configuration unit (302-1) where the aforementioned polyimide is configured with the component unit (303) and a connection unit (302-2) that connects the configuration unit (302-1). Here, the configuration unit (302-1) can include a first configuration unit for a central region and a second configuration unit for a distal region, and the first configuration unit and the second configuration unit can be connected via the connection unit (302-2). In an embodiment, the first configuration unit can include a first component unit that controls cardiac-related signals acquired from the user's body and transmits them to an external user device, and the second configuration unit can include a second component unit that directly senses and acquires cardiac-related signals from the user's body. That is, the component unit (303) can be positioned in the aforementioned configuration unit (302-1) to acquire cardiac-related signals from the human body and transmit them as an external device to the user device (400). In an embodiment, the component unit (303) can include a first component unit as a control unit that controls sensed cardiac-related signals and a second component unit as a sensing unit that actually senses cardiac-related signals, but may not be limited to the embodiment. Additionally, the signal amplification unit (304) can amplify cardiac-related signals acquired from the human body through the aforementioned copper trace and enable transmission to the user device (400). In an embodiment, the signal amplification unit (304) can further include a transceiver unit that transmits signals to the user device (400), but may not be limited to the embodiment.

Additionally, the battery unit (305) can include a battery that maintains power of the Smart All-in-One Cardiac Patch (300), which is as described above. In an embodiment, the Smart All-in-One Cardiac Patch can be a low-power device and can maintain power for an extended period through a battery. Additionally, the skin model unit (306) can be a configuration that enables the Smart All-in-One Cardiac Patch (300) to be concealed in the shape of human body skin on the outside of the housing unit (301), which is as described above. In an embodiment, the Smart All-in-One Cardiac Patch (300) can acquire at least one of ECG as an electrical signal, SCG as a low-frequency vibration signal, and PCG as an acoustic signal as cardiac-related signals and transmit them to the user device (400). Additionally, in an embodiment, the battery can perform the function of maintaining power to transmit signals acquired by the Smart All-in-One Cardiac Patch to the user device.

Thereafter, the user device (400) can analyze and display the user condition based on a condition monitoring learning model. Additionally, user condition analysis can be provided with a condition monitoring learning model that is pre-trained with cardiac-related information acquired from a plurality of user devices (400) on a cloud (500) basis, but may not be limited to the embodiment.

Additionally, in an embodiment, the Smart All-in-One Cardiac Patch System can be a system including the aforementioned Smart All-in-One Cardiac Patch (300), user device (400), and cloud (500), but may not be limited to the embodiment.

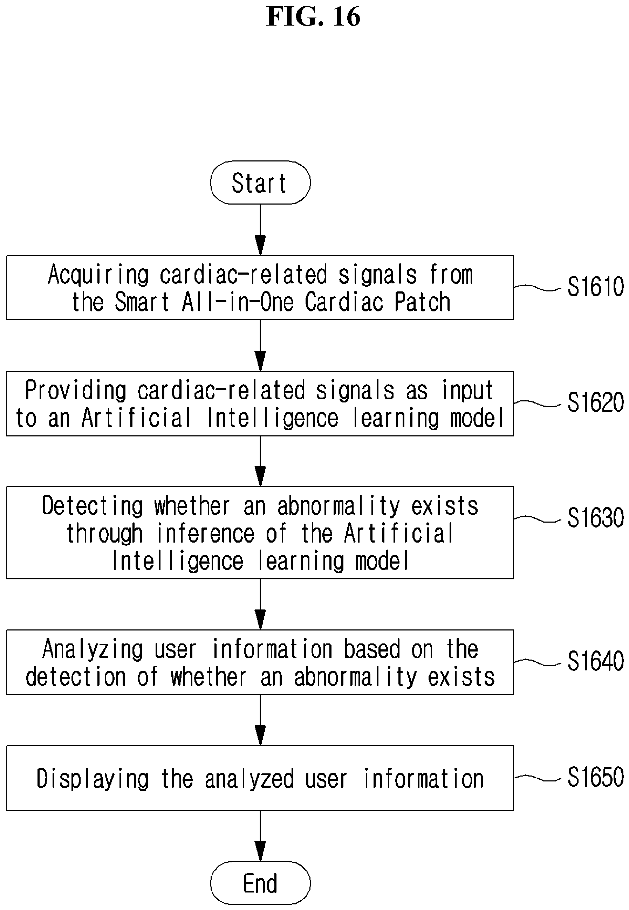

FIG. 16 is a diagram showing a method of providing cardiac-related information to a user in accordance with an embodiment of the present invention.

Referring to FIG. 16, cardiac-related signals can be acquired from a Smart All-in-One Cardiac Patch (S1610). Thereafter, the cardiac-related signals can be provided as input to a condition monitoring learning model (S1620), and whether an abnormality exists can be detected based on inference of the condition monitoring learning model (S1630). Thereafter, the user device can analyze whether an abnormality exists in the user condition based on the detected whether an abnormality exists (S1640), and can display the analyzed information and provide it to the user (S1650).

The embodiments described above are at least partially implemented as computer programs and can be recorded in a computer-readable recording medium. A program recorded for implementing the embodiments and a computer-readable recording medium include all types of storage devices in which data readable by a computer is stored. Examples of computer-readable recording media include ROM, RAM, CD-ROM, magnetic tape, optical data storage devices, and the like. Additionally, computer-readable recording media can be distributed over networked computer systems, whereby code readable by a computer can be stored and executed in a distributed manner. Furthermore, functional programs, codes, and code segments for implementing the present embodiment would be readily understood by a person having ordinary skill in the art of the present technology.

The present specification described above has been described with reference to the embodiments shown in the drawings; however, these are merely illustrative, and those having ordinary skill in the art will understand that various modifications and variations of embodiments are possible. However, such modifications should be considered within the scope of technical protection of the present specification. Accordingly, the true technical scope of protection of the present specification should be determined to include other implementations, other embodiments, and equivalents of the patent claims by the technical spirit of the appended claims.

### INDUSTRIAL APPLICABILITY

The foregoing may also be applicable to other systems.

## Claims

1. A method of providing user analysis information by a user device based on a Smart All-in-One Cardiac Patch, the method comprising:
receiving cardiac-related signals acquired from a Smart All-in-One Cardiac Patch;
providing the cardiac-related signals as input to a condition monitoring learning model;
automatically detecting whether an abnormality exists in a user based on inference of the condition monitoring learning model; and
performing user information analysis based on the detection of whether an abnormality exists in the user, and displaying the analyzed user information.

2. The method of providing user analysis information of claim 1,
wherein the condition monitoring learning model is obtained from a cloud,
wherein the cloud receives the cardiac-related signals of the Smart All-in-One Cardiac Patches respectively connected with each of a plurality of the user devices from the plurality of the user devices, and
updates the condition monitoring learning model based on a plurality of the cardiac-related signals.

3. The method of providing user analysis information of claim 2,
wherein the cloud
acquires a plurality of the cardiac-related signals in real-time,
further acquires cardiac-related raw data based on a batch load,
wherein the plurality of the cardiac-related signals acquired in real-time and the cardiac-related raw data acquired based on the batch load are processed in parallel and reflected in the condition monitoring learning model.

4. The method of providing user analysis information of claim 2,
wherein the Smart All-in-One Cardiac Patch acquires the cardiac-related signals based on user identification information and transmits them to the user device,
wherein the user device records the cardiac-related signals acquired from the Smart All-in-One Cardiac Patch together with a session key, a Medium Access Control (MAC) address-based identifier of the user device, and a unique time based on the user identification information, and
transmits cardiac-related information to the cloud based on the record, wherein the cardiac-related information is transmitted to the cloud together with a signal type, a data access time, and a data modification time based on the user identification information.

5. The method of providing user analysis information of claim 4,
wherein the cloud derives record information excluding the user identification information from information acquired from the user device, and encodes the record information with a hexadecimal key and provides it as training data for the condition monitoring learning model based on administrator authority.

6. The method of providing user analysis information of claim 1,
wherein the Smart All-in-One Cardiac Patch senses at least one of Electrocardiogram (ECG), which is an electrical signal related to cardiac beating, Seismocardiogram (SCG), which is a low-frequency vibration signal, and Phonocardiogram (PCG), which is a cardiac acoustic signal, and transmits it to the user device.

7. The method of providing user analysis information of claim 6,
wherein the user device
further acquires additional information related to the cardiac-related signals, and generates cardiac-related information based on the additional information.

8. The method of providing user analysis information of claim 1,
wherein the user device displays the analyzed user information based on an interface including user-related information and analysis information.

9. A computer program stored in a computer-readable recording medium to execute, in combination with hardware, the method of providing user analysis information according to claims 1 to 8.
